# EUROPEAN PATENT APPLICATION

(11) **EP 4 814 002 A1**
(43) Date of publication of application: **30.09.2026**
(21) Application number: 24894240.1
(22) Date of filing: 22.11.2024
(51) Int. Cl.: C07K 14/47, A61K 38/17, A61K 39/00, A61P 35/00, A61P 37/04, C12N 15/12

(54) **MODIFIED MFG-E8 PROTEIN**

(30) Priority: 24.11.2023 JP 2023199462
(71) Applicant: Tahara Hideaki c/o Osaka Prefectural Hospital Organization Osaka International Cancer Institute, Osaka-shi, Osaka 541-8567 (JP)
(72) Inventor: MIZOTE, Yu, Osaka-shi, Osaka 541-8567 (JP); TAHARA, Hideaki, Osaka-shi, Osaka 541-8567 (JP)
(74) Representative: Ipsilon Belgium
(86) International application number: PCT/JP2024/041545
(87) International publication number: WO 2025/110250

(57) **Abstract**

The present disclosure relates to a composition for cancer treatment and a method for treating cancer, a composition for activating phagocytes and a method for activating the same, a composition for activating an immune response and a method for activating the same related thereto, and a modified MFG-E8 protein or a nucleic acid encoding the same used in these compositions and methods.

## Description

### Technical Field

The present disclosure relates to a composition for cancer treatment and a method for treating cancer, a composition for activating phagocytes and a method for activating the same, a composition for activating an immune response and a method for activating the same related thereto, and a modified MFG-E8 protein or a nucleic acid encoding the same used in these compositions and methods.

### Background Art

Cancer is still the leading cause of death, and new therapeutic methods are constantly required. As a therapeutic method for cancer, there are surgical therapy, radiation therapy, chemotherapy (an anticancer agent), and immunotherapy, but treatment with an anticancer agent is used even after surgery. As the anticancer agent, an alkylating agent, an antimetabolite, an alkaloid anticancer agent, an antibiotic anticancer agent, a platinum-containing drug, and the like are used, but therapeutic effects thereof are not yet sufficient, and there is also a problem of a high frequency of side effects. From such a viewpoint, it is desired to develop superior pharmaceuticals for cancer treatment.

On the other hand, MFG-E8 (milk fat globule-EGF factor 8) was identified as a factor that is secreted from the mammary gland and promotes mammary gland differentiation and lactation stimulation (Non Patent Literature 1: Stubbs T et al. 1990. Proc. Natl Acad. Sci. USA 87:8417-8421). It has become clear that MFG-E8 has not only the above effects but also various other different functions. One of these is an important function of recognizing phosphatidylserine (PtdSer) on apoptotic cell membranes as an opsonin, promoting phagocytic processing ability by macrophages and dendritic cells, and maintaining subsequent immune tolerance (Non Patent Literature 2: Hanayama R. et al. 2002. Nature 417:182-187, Non Patent Literature 3: Hanayama R., et al. 2004. Science 304:1147-1150). Furthermore, MFG-E8 has been found to induce immune tolerance by promoting proliferation of Foxp3-positive regulatory T cells and negatively modulate anti-tumor immunity of tumor vaccines (Non Patent Literature 4: Jinushi M., et al. 2007. J Clin Invest 117:1902-1913). Based on this finding, attempts have been made to develop a therapeutic method using a decoy gene or an inhibitory antibody of MFG-E8 on the premise of combination with a tumor antigen such as a cancer vaccine (Patent Literature 1: WO 2008/043018 A). Further, it has been revealed that MFG-E8 is widely expressed not only in antigen-presenting cells such as dendritic cells but also in tumor cells such as breast cancer, colorectal cancer, and melanoma (Non Patent Literature 5: Carmon L., et al. 2002. J Clin Invest 110:453-462), has a tumor-promoting effect by promoting angiogenesis and tumor metastasis ability, and is positively correlated with clinical stage of progression of melanoma (Non Patent Literature 6: Neutzner M., et al. 2007. Cancer Res 67:6777-6785.).

The inventors of the present disclosure have previously developed a technique for enhancing the therapeutic effects of other cancer therapies using an anti-MFG-E8 antibody (Patent Literature 2: WO 2009/147781 A, Non Patent Literature 7: Jinushi and Tahara et al., JEM, 2009). The present invention utilizes the fact that phagocytosis of apoptotic cells advanced by crosslinking PtdSer and integrin by MFG-E8 is inhibited by using an antibody against integrin-binding motif RGD, and instead, phagocytosis is mediated via the Fc portion of the antibody, thereby activating a subsequent immune response, and has provided an excellent technical effect as a new method for cancer treatment. On the other hand, it has been desired to develop further new pharmaceuticals for cancer treatment from the fact that, in the same method: 1) MFG-E8 is not the only molecule that binds to PtdSer and induces immune tolerance, 2) an immune-activating effect depends on Fcγ receptors on phagocytes, and Fcγ receptors include not only those for activation but also those that suppress an immune response.

### Citation List

### Patent Literature

Patent Literature 1: WO 2008/043018 A
Patent Literature 2: WO 2009/147781 A

### Non Patent Literature

Non Patent Literature 1: Stubbs T et al. 1990. Proc. Natl. Acad. Sci. USA 87:8417-8421
Non Patent Literature 2: Hanayama R. et al. 2002. Nature 417:182-187
Non Patent Literature 3: Hanayama R., et al. 2004. Science 304:1147-1150
Non Patent Literature 4: Jinushi M., et al. 2007. J Clin. Invest. 117:1902-1913)
Non Patent Literature 5: Carmon L., et al. 2002. J Clin. Invest. 110:453-462
Non Patent Literature 6: Neutzner M., et al. 2007. Cancer Res. 67:6777-6785
Non Patent Literature 7: Jinushi and Tahara et al., JEM, 2009. 206:1317-1326

### Summary of Invention

### Technical Problem

It is still desired to develop new pharmaceuticals for cancer treatment. The present disclosure provides a new composition for cancer treatment and a method for treating cancer, a composition for activating phagocytes and a method for activating phagocytes, a composition for activating an immune response and a method for activating an immune response related thereto, and a modified MFG-E8 protein or a nucleic acid encoding the same used in these compositions and methods.

### Solution to Problem

The inventors of the present disclosure, while studying results obtained by diligently advancing research on MFG-E8, have conceived an idea of utilizing a PtdSer binding ability of MFG-E8 for a cancer treatment method, unlike conventional methods in which MFG-E8 itself is inhibited with an antibody. Through trial and error, the inventors of the present disclosure have found that while an MFG-E8 antibody is limited to inhibition of binding between MFG-E8 and integrin on phagocytes, by utilizing the PtdSer binding ability of MFG-E8, binding between PtdSer and PtdSer binding molecules other than MFG-E8 can be inhibited, thereby suppressing induction of immune tolerance. For example, regarding Gas6/protein S-TAM receptor, C1q-C1q receptor, β2-GPI, and TIM-4, which are known as PtdSer binding molecules, by inhibiting the binding between these and PtdSer, the induction of immune tolerance can be inhibited in all of them. Further, the present inventors have found that by fusing a PtdSer binding moiety for MFG-E8 with RAP (receptor-associated protein), which is a binding protein to CD91/LRP1, a phagocytosis pathway can be regulated, and the subsequent immune response can be reliably activated. Further, the present inventors have found that by using such a fusion form with the RAP, instability of the immune response caused by presence of a wide variety of Fcγ receptors observed when the MFG-E8 antibody was used can be eliminated.

In one aspect, the present disclosure provides a protein that has a PtdSer binding site of MFG-E8 and does not have an integrin αᵥβ_{3/5}, α₈β₁ binding site, that is, does not have a site at which MFG-E8 binds to integrin αᵥβ_{3/5} or integrin α₈β₁.

In one aspect, the present disclosure provides a nucleic acid encoding a protein that has a PtdSer binding site of MFG-E8 and does not have an integrin αᵥβ_{3/5}, α₈β₁ binding site.

In one aspect, the present disclosure provides a composition containing a protein that has a PtdSer binding site of MFG-E8 and does not have an integrin αᵥβ_{3/5}, α₈β₁ binding site, or a nucleic acid encoding the same.

In one aspect, the present disclosure provides a method of using a composition containing a protein that has a PtdSer binding site of MFG-E8 and does not have an integrin αᵥβ_{3/5}, α₈β₁ binding site, or a nucleic acid encoding the same.

More specifically, the present disclosure provides the following.
[Item 1]
   A protein that has a phosphatidylserine (PtdSer) binding site of milk fat globule-EGF factor 8 (MFG-E8) and does not have an integrin αᵥβ_{3/5}, α₈β₁ binding site.
[Item 2]
   The protein according to item 1, wherein the protein has a C1C2 domain of MFG-E8 or a portion thereof as a PtdSer binding site.
[Item 3]
   The protein according to item 1, wherein the protein does not have a RGD motif (Arg-Gly-Asp integrin binding motif) of MFG-E8 or a portion thereof as an integrin αᵥβ_{3/5}, α₈β₁ binding site.
[Item 4]
   The protein according to item 1, wherein the protein does not have an EGF-like domain (epidermal growth factor like domain) of MFG-E8 or a portion thereof as an integrin αᵥβ_{3/5}, α₈β₁ binding site.
[Item 5]
   The protein according to item 1, wherein the protein further has a site that binds to a receptor that is different from integrin αᵥβ_{3/5} or integrin α₈β₁ on phagocytes.
[Item 6]
   The protein according to item 5, wherein the receptor on phagocytes is a receptor that activates phagocytosis of phagocytes.
[Item 7]
   The protein according to item 6, wherein the receptor that activates phagocytosis of phagocytes is CD91/LRP1.
[Item 8]
   The protein according to item 5, wherein the site that binds to the receptor on phagocytes is a receptor-associated protein (RAP) or a portion thereof.
[Item 9]
   A nucleic acid encoding the protein according to any one of items 1 to 8.
[Item 10]
   A composition for activating phagocytosis of phagocytes, including the protein according to any one of items 1 to 8 or a nucleic acid encoding the same.
[Item 11]
   A composition for activating an immune response, including the protein according to any one of items 1 to 8 or a nucleic acid encoding the same.
[Item 12]
   A composition for treating cancer, including the protein according to any one of items 1 to 8 or a nucleic acid encoding the same.
[Item 13]
   The composition for treating cancer according to item 12, for use in combination with an anticancer therapy that exhibits a cytocidal effect.
[Item 14]
   The composition for treating cancer according to item 12, for use in combination with an anticancer therapy using an immune checkpoint inhibitor.
[Item 15]
   A method of activating phagocytosis of phagocytes, including a step of bringing a protein that has a PtdSer binding site of MFG-E8 and does not have an integrin αᵥβ_{3/5}, α₈β₁ binding site, or a nucleic acid encoding the same, into contact with PtdSer of apoptotic cells.
[Item 16]
   A method of activating phagocytosis of phagocytes in a subject having a disease, symptom, or condition requiring activation of phagocytosis of phagocytes, including a step of administering to the subject a composition containing a protein that has a PtdSer binding site of MFG-E8 and does not have an integrin αᵥβ_{3/5}, α₈β₁ binding site, or a nucleic acid encoding the same.
[Item 17]
   The method according to item 15 or 16, wherein the protein has a C1C2 domain of MFG-E8 or a portion thereof as the PtdSer binding site.
[Item 18]
   The method according to item 15 or 16, wherein the protein does not have the RGD motif (Arg-Gly-Asp integrin binding motif) of MFG-E8 or a portion thereof as the integrin αᵥβ_{3/5}, α₈β₁ binding site.
[Item 19]
   The method according to item 15 or 16, wherein the protein does not have an EGF-like domain (epidermal growth factor like domain) of MFG-E8 or a portion thereof as the integrin αᵥβ_{3/5}, α₈β₁ binding site.
[Item 20]
   The method according to Item 15 or 16, wherein the protein further has a site that binds to a receptor that is different from integrin αᵥβ_{3/5} or integrin α₈β₁ on phagocytes.
[Item 21]
   The method according to item 15 or 16, wherein the receptor on phagocytes is a receptor that activates phagocytosis of phagocytes.
[Item 22]
   The method according to item 15 or 16, wherein the receptor that activates phagocytosis of phagocytes is CD91/LRP1.
[Item 23]
   The method according to item 15 or 16, wherein the site that binds to the receptor on phagocytes is a receptor-associated protein (RAP) or a portion thereof.
[Item 23]
   The method according to item 16, wherein the disease, symptom, or condition requiring activation of phagocytosis of phagocytes is cancer.
[Item 28]
   The method according to item 23, for use in combination with an anticancer therapy that exhibits a cytocidal effect.
[Item 29]
   The method according to Item 23, for use in combination with an anticancer therapy using an immune checkpoint inhibitor.

### Advantageous Effects of Invention

The present disclosure has an effect of providing a new composition for cancer treatment and a method for treating cancer, a composition for activating phagocytes and a method for activating the same, a composition for activating an immune response and a method for activating the same related thereto, and a modified MFG-E8 protein or a nucleic acid encoding the same used in these compositions and methods.

### Brief Description of Drawings

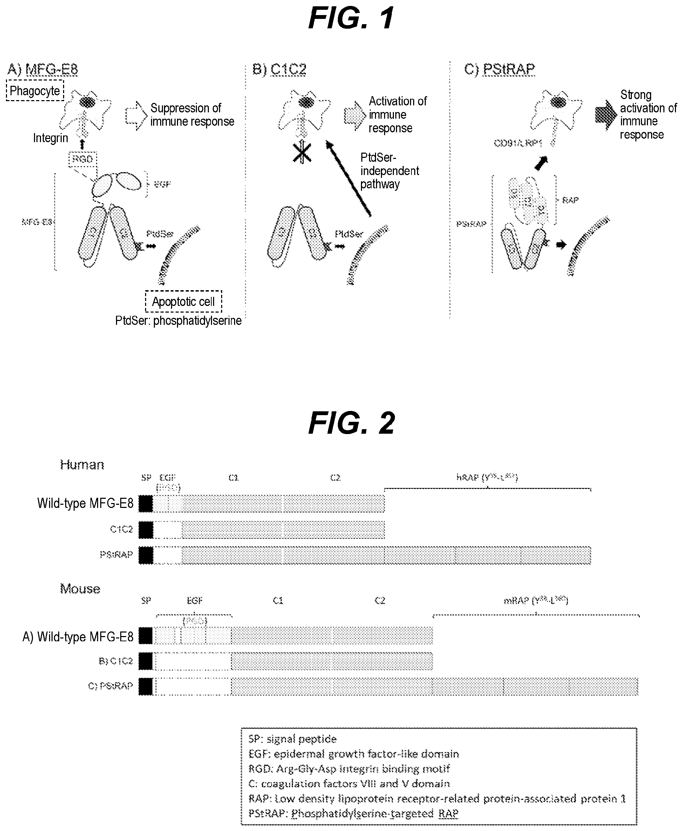

Fig. 1 is a schematic diagram illustrating a mechanism of action of the present disclosure. (A) In normal cells, MFG-E8 recognizes PtdSer on an apoptotic cell membrane and binds to integrins αᵥβ_{3/5} and α₈β₁ on phagocytes, thereby promoting phagocytosis of phagocytes and suppressing a subsequent immune response. (B) A protein that has a PtdSer binding site and does not have an integrin αᵥβ_{3/5}, α₈β₁ binding site according to the present disclosure restricts an immunosuppressive phagocytic pathway of phagocytes by inhibiting binding of MFG-E8 and other PtdSer-binding phagocytosis-promoting factors to PtdSer. As a result, phagocytes activated by a PtdSer-independent pathway can activate the subsequent immune response. (C) By further fusing RAP, which binds to a CD91/LRP1 receptor on phagocytes, to the protein of the present disclosure, the phagocytic pathway is regulated and induced toward one favorable for inducing an immune response, and a subsequent immune response is strongly activated.
Fig. 2 is a schematic diagram illustrating composition examples of a modified human MFG-E8 protein or a modified mouse MFG-E8 protein of the present disclosure. Wild-type MFG-E8 (A) shows the composition of a wild-type mouse MFG-E8 protein. C1C2 (B) shows the composition of a modified mouse MFG-E8 protein with a deletion of an EGF-like domain. PStRAP (C) shows the composition of a modified MFG-E8 protein in which a portion of RAP (Y38 to L360) is fused to a C-terminus of the modified mouse MFG-E8 protein with a deletion of the EGF-like domain.
Fig. 3 is a diagram illustrating results of an experiment confirming anticancer effects of C1C2 and PStRAP of a modified MFG-E8 protein of the present disclosure.
Fig. 4 is a diagram illustrating results of an experiment confirming properties of LNP.
Fig. 5 is a diagram illustrating results of evaluating liver dysfunction caused by LNP administration.
Fig. 6 is a diagram illustrating results of evaluating binding specificity of PStRAP to cell membrane lipids.
Fig. 7 is a diagram illustrating measurement results of an amount of PStRAP in serum after LNP-PStRAP administration.
Fig. 8 is a diagram illustrating an outline of an experimental method for evaluating liver dysfunction caused by LNP administration.
Fig. 9 is a diagram illustrating results of an experiment for evaluating liver dysfunction caused by LNP administration.

### Description of Embodiments

Usually, removal of apoptotic cells is carried out without inflammation because it suppresses the immune response against autologous cells, and apoptotic cells are rapidly digested by phagocytes (avoidance of autoimmune diseases). Conversely, however, the immune response against cancer cells where a boundary between self and non-self is ambiguous is also suppressed. A suppression mechanism accompanying this removal of apoptotic cells is based on the binding of factors such as MFG-E8, Gas6, β2-GPI, and C1q to PtdSer exposed on the apoptotic cell membrane. The present disclosure focuses on utilizing the C1C2 domain, which is a PtdSer-binding domain of MFG-E8, and its basic operation principle is to cover PtdSer with a molecule in which an integrin binding domain has been deleted from the C1C2 domain and to inhibit recognition of the PtdSer-binding molecule (Fig. 1, Fig. 2A, B). Furthermore, a fusion protein (PStRAP; PtdSer-targeted RAP) in which RAP that is a binding protein to CD91/LRP1 is bound to the C1C2 domain was utilized to control a phagocytic method of phagocytes (Fig. 1, Fig. 2C). CD91/LRP1 is also a common receptor of many heat shock proteins in addition to RAP, and phagocytosis mediated by CD91/LRP1 is known to excite strong immune induction.

As a method for activating an immune response by dead cells in vivo, the concept of "in situ vaccination" is being studied worldwide. However, most of the studies focus on "how to kill" cancer cells, which has been systematized in the form of immunogenic cell death. On the other hand, there are few studies focusing on "how to process (phagocytose)" regardless of the method of killing. The related application by the present inventors (Japanese Patent Application No. 2010-515738, WO 2009/147781 A) is a pioneering study of this strategy. The present disclosure has been completed by focusing on phagocytosis of cancer cells, and compared to the above related applications of the inventors of the present disclosure, has a broader range of applications, can be used in combination with almost all standard anticancer therapies except surgical treatments, and is expected to enhance therapeutic effects.

Further, the immune checkpoint inhibitor recognized as a new standard therapy shares the same strategy of "activating an anticancer immune response", but conventional immune checkpoint inhibitors take release of suppression of immune cells after activation as a therapeutic strategy, and thus have a different point of action from the present disclosure that activates induction of an immune response. Therefore, it is considered that the composition for treating cancer and the method for treating cancer according to the present disclosure are also effective as a concomitant drug with an immune checkpoint inhibitor.

### (Definitions)

Unless defined otherwise, all technical and scientific terms used in the present description have the same meaning as commonly understood by one of ordinary skill in the art to which the present disclosure belongs.

In the present description, in a case where a plurality of numerical ranges are indicated, a range consisting of any combination of lower limit and upper limit of the plurality of ranges is also meant in the same manner.

The term "substantially" in the present description has the same meaning as commonly understood by one of ordinary skill in the art to which the present disclosure belongs, but is used with an intention to encompass, for example, an intended state and states that are not unavoidably achieved due to biological or chemical properties, taking into account that a biological or chemical phenomena may not completely achieve the intended state.

In the present description, in a case where the term "about" is used in relation to a numerical value, it means that the value may vary within the range of, for example, 10%, 9%, 8%, 7%, 6%, 5%, 4%, 3%, 2%, 1%, 0.5%, 0.1%, 0.05%, or 0.01%.

In the present description, the terms "include" or "contain" have the same meaning as commonly understood by one of ordinary skill in the art to which the present disclosure belongs, but encompass, for example, "including" and "consisting of", and specifically, a composition "including" or "containing" A may include another component, B, in addition to including A alone.

In the present description, the term "have" has the same meaning as commonly understood by those skilled in the art to which the present disclosure belongs, but has a meaning similar to, for example, the above "include" or "contain", and a protein "having" A may have another component, B, in addition to having A alone.

In the present description, the terms "consisting of" or "composed of" as used in reference to a composition have the same meaning as commonly understood by one of ordinary skill in the art to which the present disclosure belongs, but are used to indicate components that exclusively constitute the composition. For example, a composition "consisting of" A exclusively contains A alone. However, in one aspect, a composition "consisting of" A encompasses aspects that include contaminants other than A that are unavoidable in manufacturing based on biological and chemical properties.

In the present description, the term "identity" refers to a degree of identical amino acid sequences or base sequences between two or more comparable amino acid sequences or base sequences. Therefore, the higher the identity between two amino acid sequences or base sequences, the higher the identity or similarity of those sequences. A level of identity of amino acid sequences or base sequences is usually determined using FASTA, a tool for sequence analysis, with default parameters. Alternatively, it can be determined using the BLAST algorithm by Karlin and Altschul (for example, Karlin S, Altschul SF. Proc. Natl Acad Sci USA. 87:2264-2268 (1990), Karlin S, Altschul SF. Natl Acad Sci USA. 90:5873-7 (1993), etc.). Programs called BLASTN and BLASTX based on such a BLAST algorithm have been developed (for example, Altschul SF, GishW, Miller W, Myers EW, Lipman DJ. J Mol Biol. 215:403-10 (1990), etc.). Specific techniques for these analysis methods are known and can be referred to on the NCBI website. For example, a certain amino acid sequence A being a specific percentage identical to another amino acid sequence B means that the amino acid sequence A and the amino acid sequence B have the percentage of identity.

In the present description, the term "conservative substitution" means that an amino acid residue is substituted with an amino acid residue having a similar side chain.

For example, substitution between amino acid residues having basic side chains such as lysine, arginine, and histidine corresponds to conservative substitution. Similarly, other substitutions between amino acid residues having acidic side chains, such as aspartic acid and glutamic acid; amino acid residues having non-charged polar side chains, such as glycine, asparagine, glutamine, serine, threonine, tyrosine, and cysteine; amino acid residues having non-polar side chains, such as alanine, valine, leucine, isoleucine, proline, phenylalanine, methionine, or tryptophan; amino acid residues having β-branched side chains, such as threonine, valine, and isoleucine; and amino acid residues having aromatic side chains, such as tyrosine, phenylalanine, tryptophan, and histidine, are also conservative substitutions.

In the present description, "treatment" or "therapy" of a disease, symptom, or condition has the same meaning as commonly understood by one of ordinary skill in the art to which the present disclosure belongs, but encompasses any act beneficial in a subject having the disease, symptom, or condition. In one aspect, "treatment" or "therapy" of a disease, symptom, or condition includes suppression, arrest of progression, slowing of progression, amelioration, and prevention of the disease, symptom, or condition.

In the present description, "activation" of a physiological activity such as phagocytosis of phagocytes or an immune response has the same meaning as commonly understood by those skilled in the art to which the present disclosure belongs, but encompasses any aspect that induces a state in which the physiological activity is more effectively exerted, and includes inducing the activity from a situation where the physiological activity is absent and enhancing an already existing physiological activity.

### (Protein having PtdSer binding site of MFG-E8 but not having integrin αᵥβ_{3/5}, α₈β₁ binding site)

In one aspect, the present disclosure provides a protein that has a PtdSer binding site of MFG-E8 and does not have an integrin αᵥβ_{3/5}, α₈β₁ binding site. PtdSer normally presented on the surface of apoptotic cells is recognized by factors such as MFG-E8, Gas6, β2-GPI, C1q, and TIM-4, and causes phagocytosis of phagocytes to promote rapid digestion of apoptotic cells and suppress a subsequent immune response. On the other hand, the protein of the present disclosure binds to PtdSer presented on the surface of apoptotic cells by the PtdSer binding site of MFG-E8, thereby inhibiting the binding between PtdSer and factors such as MFG-E8, Gas6, β2-GPI, C1q, and TIM-4, and consequently activates the subsequent immune response by regulating the phagocytic pathway of phagocytes.

In one aspect, the PtdSer binding site of MFG-E8 included in the protein of the present disclosure is an amino acid sequence contained in MFG-E8 and has a sequence region necessary and sufficient for binding to PtdSer. The sequence region can be identified by those skilled in the art based on the literature or by carrying out experiments common to those skilled in the art.

In one aspect, the PtdSer binding site of MFG-E8 included in the protein of the present disclosure has a full length or a portion of the C1C2 domain of coagulation factors VIII and V-type C domains. The C1C2 domain of MFG-E8 is well known to those skilled in the art, but for example, in a case where MFG-E8 is human MFG-E8 (NCBI Gene ID: 4240), the C1C2 domain is identified as a region of amino acid sequence 70 to 387.

In one aspect, the PtdSer binding site of MFG-E8 included in the protein of the present disclosure may have a mutation in the amino acid sequence of wild-type MFG-E8 as long as the ability to bind to PtdSer is maintained. For example, the PtdSer binding site of MFG-E8 included in the protein of the present disclosure may have a conservative substitution for the amino acid sequence of wild-type MFG-E8. In one aspect, the PtdSer binding site of MFG-E8 included in the protein of the present disclosure has at least about 80%, at least about 85%, at least about 90%, at least about 95%, at least about 98%, or at least about 99% sequence identity to the amino acid sequence of wild-type MFG-E8 at the site. In one aspect, the protein of the present disclosure contains a portion of MFG-E8. In one aspect, the protein of the present disclosure consists substantially of a portion of MFG-E8.

The protein of the present disclosure does not have an integrin αᵥβ_{3/5}, α₈β₁ binding site. The site is an amino acid sequence contained in MFG-E8, and is a sequence region necessary for binding to integrins αᵥβ_{3/5} and α₈β₁. The sequence region can be identified by those skilled in the art based on the literature or by carrying out experiments common to those skilled in the art.

In one aspect, the protein of the present disclosure does not have the full length or a portion of the RGD motif (Arg-Gly-Asp integrin motif) as the integrin αᵥβ_{3/5}, α₈β₁ binding site. The RGD motif of MFG-E8 is well known to those of skill in the art, but for example, in a case where MFG-E8 is human MFG-E8 (NCBI Gene ID: 4240), the RGD motif is identified as the amino acid sequence arginine-glycine-aspartic acid contained in the region of amino acid sequence 46 to 48.
>Amino acid sequence of human MFG-E8 (NCBI Gene ID: 4240) (SEQ ID NO: 3)

MPRPRLLAALCGALLCAPSLLVALDICSKNPCHNGGLCEEISQEVRGDVFPSYTCTCLKGYAGNHCETKC
VEPLGLENGNIANSQIAASSVRVTFLGLQHWVPELARLNRAGMVNAWTPSSNDDNPWIQVNLLRRMWVTG
VVTQGASRLASHEYLKAFKVAYSLNGHEFDFIHDVNKKHKEFVGNWNKNAVHVNLFETPVEAQYVRLYPT
SCHTACTLRFELLGCELNGCANPLGLKNNSIPDKQITASSSYKTWGLHLFSWNPSYARLDKQGNFNAWVA
GSYGNDQWLQVDLGSSKEVTGIITQGARNFGSVQFVASYKVAYSNDSANWTEYQDPRTGSSKIFPGNWDN
HSHKKNLFETPILARYVRILPVAWHNRIALRLELLGC

In one aspect, the protein of the present disclosure does not have the full length or a portion of the EGF-like domain (epidermal growth factor like domain) as the integrin αᵥβ_{3/5}, α₈β₁ binding site. The EGF-like domain of MFG-E8 is well known to those of skill in the art, but for example, in a case where MFG-E8 is human MFG-E8 (NCBI Gene ID: 4240), the EGF-like domain is identified as the region of amino acid sequence 24 to 67.

In one aspect, the protein of the present disclosure further has a site that binds to a receptor on phagocytes. In one aspect, the receptor on phagocytes is not integrin αᵥβ_{3/5} or α₈β₁. In one aspect, the receptor on phagocytes is not integrin αᵥβ_{3/5}, α₈β₁, or a TAM receptor (Tyro3, Axl, Mer). In one aspect, the protein of the present disclosure is provided as a fusion protein of a portion of MFG-E8 and a site that binds to a receptor on phagocytes. In one aspect, the protein of the present disclosure may contain a signal peptide of MFG-E8.

In one aspect, the receptor on phagocytes is a receptor that activates phagocytosis of phagocytes. The receptor that activates the phagocytosis of phagocytes includes a receptor having an action of inducing phagocytosis in phagocytes that do not exhibit phagocytosis, and a receptor that enhances phagocytosis in phagocytes that exhibit phagocytosis.

The receptor that activates the phagocytosis of phagocytes is not particularly limited, and a receptor known to those skilled in the art can be appropriately selected as a receptor that activates phagocytosis of phagocytes. For example, the receptor is selected from, but not limited to, CD91/LRP, an Fc receptor, and a complement receptor (CR1, CR3, CR4).

An additional site that binds to a receptor on phagocytes included in the protein of the present disclosure is not particularly limited, and a compound known to those skilled in the art can be appropriately selected as a site that binds to a receptor that activates phagocytosis of phagocytes. A structure of the compound is not particularly limited, and a compound such as a protein, a peptide, and a nucleic acid can be used. For example, a receptor-associated protein (RAP), heat shock proteins (Hsp70, Hsp90, gp96, calreticulin), the Fc region of an antibody, C3b, iC3b, or portions thereof can be utilized.

In a case where a RAP is used in the present disclosure, the full length of the RAP may be used, or a portion thereof that binds to a receptor on phagocytes may be used. In one aspect, the region of the RAP used in the present disclosure is not particularly limited as long as it maintains the ability to activate phagocytosis of phagocytes. In one aspect, the RAP used in the present disclosure preferably includes D1 to D3 domains. For example, in a case where human RAP (NCBI Gene ID: 4043) is used, it is preferable to use a portion including the region of Y35 to L357. In a case where mouse RAP (NCBI Gene ID: 16976) is used, it is preferable to use a portion including the regions of Y38 to L360. In one aspect, the RAP used in the present disclosure includes the D3 domain (R237 to R353 of human RAP and R240 to R356 of mouse RAP) and does not include D1 and D2. In one aspect, the RAP used in the present disclosure substantially includes only a portion necessary to maintain the ability to activate phagocytosis of phagocytes. For example, the RAP used in the present disclosure does not include an endoplasmic reticulum retention sequence (Bu G. et al., EMBO J. 1995; 14(10): 2269-80) consisting of the C-terminal four amino acids (HNEL).

In one aspect, the RAP in the protein of the present disclosure may have a mutation in the amino acid sequence of wild-type RAP as long as the ability to activate phagocytosis of phagocytes is maintained. For example, the RAP in the protein of the present disclosure may have conservative substitutions relative to the amino acid sequence of wild-type RAP. In one aspect, the RAP in the protein of the present disclosure has at least about 80%, at least about 85%, at least about 90%, at least about 95%, at least about 98%, or at least about 99% sequence identity to the amino acid sequence of wild-type RAP.

An additional domain that binds to a receptor on phagocytes included in the protein of the present disclosure can be bound to the PtdSer binding domain directly or by using a linker. In one aspect, the protein having the additional domain that binds to a receptor on phagocytes is provided as a fusion protein.

The phagocytes are not particularly limited, and cells known to those skilled in the art can be appropriately selected as cells exhibiting phagocytosis. In one aspect, the phagocytes are cells that activate the immune response through phagocytosis. For example, monocytes, macrophages, and dendritic cells can be selected as the phagocytes, but the phagocytes are not limited thereto.

An MFG-E8-derived portion, or an additional site that binds to a receptor on phagocytes included in the protein of the present disclosure that has a PtdSer binding site of MFG-E8 and does not have an integrin αᵥβ_{3/5}, α₈β₁ binding site may be derived from proteins of various organisms. For example, it may be derived from organisms such as primates such as a human or a monkey, or mammals such as a dog, a cat, or a mouse.

The protein of the present disclosure that has a PtdSer binding site of MFG-E8 and does not have an integrin αᵥβ_{3/5}, α₈β₁ binding site can have additional modifications. For example, it may be modified with various compounds for purposes such as improving storage stability, controlling blood retention time, and the like. Examples of such compounds include, but are not limited to, PEG (polyethylene glycol). The protein of the present disclosure can include sites with other functions in addition to the PtdSer binding site and the site that binds to a receptor on phagocytes.

### (Nucleic acid encoding protein having PtdSer binding site of MFG-E8 but not having integrin αᵥβ_{3/5}, α₈β₁ binding site)

The present disclosure, in one aspect, provides a nucleic acid encoding a protein that has a PtdSer binding site of MFG-E8 and does not have an integrin αᵥβ_{3/5}, α₈β₁ binding site. Typically, the protein is the protein described above (protein that has a PtdSer binding site of MFG-E8 and does not have an integrin αᵥβ_{3/5}, α₈β₁ binding site). In one aspect, the nucleic acid of the present disclosure encodes substantially a portion of MFG-E8 or a fusion protein of a portion of MFG-E8 and a site that binds to a receptor on phagocytes.

The nucleic acid of the present disclosure may be ribonucleotides or deoxynucleotides. Further, the form of the nucleic acid is not particularly limited, and may be a single-stranded form or a double-stranded form. Codons used in the nucleic acid sequence are not particularly limited, and various codons can be appropriately selected and used depending on the purpose. For example, it can be appropriately selected in consideration of codon frequency and the like depending on the type of host cell, expression system, and the like adopted when producing the protein.

The nucleic acid of the present disclosure can include any additional moiety for controlling transcription, replication, and the like of the nucleic acid in addition to a moiety encoding a protein that has a PtdSer binding site of MFG-E8 and does not have an integrin αᵥβ_{3/5}, α₈β₁ binding site. In one aspect, the nucleic acid of the present disclosure is provided as a vector.

In one aspect, the nucleic acid of the present disclosure is used to express and produce the protein according to the present disclosure. In one aspect, the nucleic acids of the present disclosure are recombinantly introduced into host cells, such as E. coli cells, simian COS cells, Chinese hamster ovary (CHO) cells, human embryonic kidney (HEK293) cells, in the form of DNA, and utilized to produce the proteins of the present disclosure from the recombinant host cells.

In one aspect, the nucleic acid of the present disclosure is used to express the protein according to the present disclosure in vivo and induce a physiological effect in vivo. In one aspect, the nucleic acid of the present disclosure is delivered into a living body by various drug delivery systems in the form of DNA or mRNA, and is utilized to express the protein of the present disclosure at a desired location in the living body and at a desired time.

### (Composition containing protein having PtdSer binding site of MFG-E8 but not having integrin αᵥβ_{3/5}, α₈β₁ binding site, or nucleic acid encoding the same)

In one aspect, the present disclosure provides a composition containing a protein that has a PtdSer binding site of MFG-E8 and does not have an integrin αᵥβ_{3/5}, α₈β₁ binding site, or a nucleic acid encoding the same. Typically, the composition of the present disclosure includes the protein described above (protein that has a PtdSer binding site of MFG-E8 and does not have an integrin αᵥβ_{3/5}, α₈β₁ binding site), or the protein or the nucleic acid described above (nucleic acid encoding a protein that has a PtdSer binding site of MFG-E8 and does not have an integrin αᵥβ_{3/5}, α₈β₁ binding site).

In one aspect, the composition of the present disclosure is used to activate phagocytosis of phagocytes. Therefore, in one aspect, the present disclosure provides a composition for activating phagocytosis of phagocytes, containing a protein that has a PtdSer binding site of MFG-E8 and does not have an integrin αᵥβ_{3/5}, α₈β₁ binding site, or a nucleic acid encoding the same.

In one aspect, the composition for activating phagocytosis of phagocytes of the present disclosure can be used to activate phagocytosis of phagocytes in vivo or in vitro. In one aspect, the composition for activating phagocytosis of phagocytes of the present disclosure can be used as a reagent for analyzing the function of phagocytes in vivo or in vitro.

In one aspect, the composition for activating phagocytosis of phagocytes of the present disclosure is administered to activate phagocytosis of phagocytes in a subject having a disease, symptom, or condition requiring activation of phagocytosis of phagocytes.

In one aspect, the composition for activating phagocytosis of phagocytes of the present disclosure is used to treat a disease, symptom, or condition in a subject having the disease, symptom, or condition requiring activation of phagocytosis of phagocytes.

In one aspect, the composition for activating phagocytosis of phagocytes of the present disclosure is used as a composition for activating an immune response or a composition for treating cancer, which are described below.

In one aspect, the compositions of the present disclosure are used to activate an immune response.

Therefore, in one aspect, the present disclosure provides a composition for activating an immune response, containing a protein that has a PtdSer binding site of MFG-E8 and does not have an integrin αᵥβ_{3/5}, α₈β₁ binding site, or a nucleic acid encoding the same.

In one aspect, the composition for activating an immune response of the present disclosure is used to activate an immune response in a subject having a disease, symptom, or condition requiring activation of an immune response.

In one aspect, the composition for activating an immune response of the present disclosure is used to treat a disease, symptom, or condition in a subject having the disease, symptom, or condition requiring activation of an immune response.

In one aspect, the composition for activating an immune response of the present disclosure is used as a composition for treating cancer, as described below.

In one aspect, the composition of the present disclosure is used to treat cancer. Therefore, in one aspect, the present disclosure provides a composition for treating cancer, containing a protein that has a PtdSer binding site of MFG-E8 and does not have an integrin αᵥβ_{3/5}, α₈β₁ binding site, or a nucleic acid encoding the same.

The composition for treating cancer of the present disclosure can be used in combination with another cancer treatment method as long as its effect is not lost. The other cancer treatment method is not particularly limited, and a known treatment method known to those skilled in the art as a cancer therapy can be used. Examples of the treatment method include, but are not limited to, radiation therapy (including gamma knife therapy, cyberknife therapy, boron neutron capture therapy, proton beam therapy/heavy particle beam therapy), MR-guided focused ultrasound surgery, cryotherapy, radiofrequency ablation, ethanol injection therapy, and arterial embolization in addition to surgery.

A cancer therapeutic agent used in the cancer treatment method used in combination with the composition for treating cancer of the present disclosure is not particularly limited. For example, non-limiting examples of the cancer therapeutic agent include an alkylating agent, an antimetabolite, a microtubule inhibitor, an antibiotic anticancer agent, a topoisomerase inhibitor, a platinum-containing drug, a molecularly targeted drug, a hormone agent, and biologics. Examples of the alkylating agent include cyclophosphamide, ifosfamide, nitrosourea, dacarbazine, temozolomide, nimustine, busulfan, melphalan, procarbazine, and ranimustine. Examples of the antimetabolite include enocitabine, carmofur, capecitabine, tegafur, tegafur-uracil, tegafur-gimeracil-oteracil potassium, gemcitabine, cytarabine, cytarabine ocfosfate, nelarabine, fluorouracil, fludarabine, pemetrexed, pentostatin, methotrexate, cladribine, doxyfluridine, hydroxycarbamide, and mercaptopurine. Examples of the microtubule inhibitor include alkaloid-based anticancer agents such as vincristine, and taxane-based anticancer agents such as docetaxel and paclitaxel. Examples of the antibiotic anticancer agent include mitomycin C, doxorubicin, epirubicin, daunorubicin, bleomycin, actinomycin D, aclarubicin, idarubicin, pirarubicin, peplomycin, mitoxantrone, amrubicin, and zinostatin stimalamer. Examples of the topoisomerase inhibitor include CPT-11, irinotecan, nogitecan, which have a topoisomerase I inhibitory action, and etoposide and sobuzoxane, which have a topoisomerase II inhibitory action. Examples of the platinum-containing drug include cisplatin, nedaplatin, oxaliplatin, and carboplatin. Examples of the hormone agent include dexamethasone, finasteride, tamoxifen, anastrozole, exemestane, ethinyl estradiol, chlormadinone, goserelin, bicalutamide, flutamide, prednisolone, leuprorelin, letrozole, estramustine, toremifene, phosphestrol, mitotane, methyltestosterone, medroxyprogesterone, and mepitiostane. Examples of the biologics include interferons α, β, and γ, interleukin 2, ubenimex, and dried BCG. Examples of the molecularly targeted drug include rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tocilizumab, bortezomib, gemtuzumab ozogamicin, ibritumomab ozogamicin, ibritumomab tiuxetan, tamibarotene and tretinoin. Further, other examples include molecularly targeted drugs such as inhibitors targeting angiogenesis, such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and vascular endothelial growth factor receptor 2 inhibitors (α-VEGFR-2 antibody), various tyrosine kinase inhibitors such as MAP kinase inhibitors, inhibitors targeting cytokines, proteasome inhibitors, and antibody-anticancer agent combinations, and complement-targeted drugs. These inhibitors also include antibodies. Further, the cancer therapeutic agent may be used in combination with the following drugs: thalidomide, everolimus, elplat, ABI-007, ixabepilone, miriplatin, lapatinib, pemetrexed, cladribine, liposomal doxorubicin, Z-100, hycamtin, vandetanib, ZD4054, anastrozole, GSK1572932A, pazopanib, denosumab, S-1, motesanib, trastuzumab, Enzastaurin, imusist, NIK-333, axitinib, bosutinib, E7080, sobridotin, degarelix, fulvestrant, zoladex, cediranib, eribulin, TSU-68, TAC-101, TAS-108, NK911, NK105, erlotinib, LBH589, MK-0457, tamibarotene, lenalidomide, BNP1350, AZD0530, AZD1152, AZD2281, AZD4877, ABT-869, ONO-4538, OTS102, KW-0761, ARQ197, ofatumumab, AMG655, TAK-700, TAK-683, TAK-448, CBP501, TAK-285, TAK-593, MLN8054, MLN4924, pertuzumab, R1507, NK012, BIBF1120, BIBW2992, Patupilone, MK-2461, CP751,871, PF-00299804, satraplatin, CMC-544, YM155, GPI21016, YHO-13351, mogamulizumab, secukinumab, eculizumab, ravulizumab, avacopan, and Pegcetacoplan.

In one aspect, the composition for treating cancer of the present disclosure is preferably used in combination with another cancer therapy exhibiting a cytocidal effect. When used in combination with the other cancer therapy exhibiting a cytocidal effect, the composition for treating cancer of the present disclosure can induce an acquired immune response associated with cancer cell killing and enhance a cancer therapeutic effect.

The other cancer therapy exhibiting a cytocidal effect is not particularly limited, and a known treatment method known as a cancer therapy exhibiting a cytocidal effect to those skilled in the art can be used. For example, as a cancer therapy exhibiting a cytocidal effect, chemotherapy, radiation therapy, CAR-T therapy, oncolytic virus therapy, and the like can be used, but are not limited thereto. In one aspect, as a drug to be used in a cancer therapy exhibiting a cytocidal effect, an alkylating agent, an antimetabolite, a microtubule inhibitor, an antibiotic anticancer agent, a topoisomerase inhibitor, a platinum-containing drug, a molecularly targeted drug, and the like characterized by cytotoxic activity are particularly preferred, but are not limited thereto. Specifically, gemcitabine, 5-FU, CPT-11, etoposide, cisplatin, oxaliplatin, paclitaxel, docetaxel, dacarbazine, doxorubicin, bevacizumab, cetuximab, an anti-vascular endothelial growth factor receptor 2 inhibitory antibody, an epidermal growth factor tyrosine kinase inhibitor, and the like can be used, but are not limited thereto.

In a case where the composition for treating cancer of the present disclosure is used in combination with another cancer therapy exhibiting a cytocidal effect, a timing of administration of the composition for treating cancer of the present disclosure and a timing of use of the other cancer therapy exhibiting a cytocidal effect can be appropriately combined. In one aspect, the composition for treating cancer of the present disclosure is used simultaneously with the other cancer therapy exhibiting a cytocidal effect. In one aspect, the composition for treating cancer of the present disclosure is used before the other cancer therapy exhibiting a cytocidal effect. In one aspect, the composition for treating cancer of the present disclosure is used after the other cancer therapy exhibiting a cytocidal effect.

In one aspect, the composition for treating cancer of the present disclosure is preferably used in combination with a cancer treatment method using an immune checkpoint inhibitor. Since the composition for treating cancer of the present disclosure enhances induction of an acquired immune response, the effect of an immune checkpoint inhibitor that releases control of the immune response after induction and activation can be enhanced. The immune checkpoint inhibitor is not particularly limited, and a known agent known to those skilled in the art as an immune checkpoint inhibitor can be used. For example, as an immune checkpoint inhibitor, an anti-CTLA-4 antibody, a PD1 blocker, a PDL1 blocker, a LAG-3 inhibitor, a B7-H3 inhibitor, a B7-H4 inhibitor, a TIM3 inhibitor, and the like can be used, but are not limited thereto. In one aspect, as an anti-CTLA-4 antibody, ipilimumab and tremelimumab can be used, but are not limited thereto. In one aspect, as a PD1 blocker, nivolumab, lambrolizumab, cemiplimab, CT-011, and AMP-224 can be used, but are not limited thereto. In one aspect, as a PDL1 blocker, durvalumab, avelumab, atezolizumab, BMS-936559, and FAZ053 can be used, but are not limited thereto. In one aspect, as a LAG-3 inhibitor, IMP321 can be used, but is not limited thereto. In one aspect, as a B7-H3 inhibitor, MGA271 can be used, but is not limited thereto.

In a case where the composition for treating cancer of the present disclosure is used in combination with a method for treating cancer using an immune checkpoint inhibitor, the timing of administration of the composition for treating cancer of the present disclosure and the timing of use of the method for treating cancer using an immune checkpoint inhibitor can be appropriately combined. In one aspect, the composition for treating cancer of the present disclosure is used simultaneously with the method for treating cancer using an immune checkpoint inhibitor. In one aspect, the composition for treating cancer of the present disclosure is used before the method of treating cancer using an immune checkpoint inhibitor. In one aspect, the composition for treating cancer of the present disclosure is used after the method of treating cancer using an immune checkpoint inhibitor.

The composition for treating cancer of the present disclosure is effective against a wide variety of cancers. Cancers for which the composition for treating cancer of the present disclosure is used include, but are not limited to, for example, epithelial cancers such as pharyngeal cancer, laryngeal cancer, tongue cancer, lung cancer, breast cancer, esophageal cancer, gastric cancer, colorectal cancer, uterine cancer, ovarian cancer, liver cancer, pancreatic cancer, gallbladder cancer, kidney cancer, prostate cancer, malignant melanoma, and thyroid cancer, and non-epithelial cancers such as osteosarcoma, chondrosarcoma, rhabdomyosarcoma, leiomyosarcoma, liposarcoma, angiosarcoma, fibrosarcoma, leukemia, malignant lymphoma, and myeloma.

The composition for activating phagocytosis of phagocytes and the composition for treating cancer of the present disclosure can be formulated by mixing, dissolving, granulating, tableting, emulsifying, encapsulating, lyophilizing, and the like, together with a pharmaceutically acceptable carrier well known in the art.

For oral administration, the protein or nucleic acid of the present disclosure can be formulated into a dosage form such as a tablet, a pill, a sugar-coated tablet, a soft capsule, a hard capsule, a solution, a suspension, an emulsion, a gel, a syrup, and a slurry together with a pharmaceutically acceptable solvent, an excipient, a binder, a stabilizer, a dispersant, or the like.

For parenteral administration, the protein or nucleic acid of the present disclosure can be formulated in a dosage form such as an injection solution, a suspension, an emulsion, a cream, an ointment, an inhalant, or a suppository together with a pharmaceutically acceptable solvent, an excipient, a binder, a stabilizer, a dispersant, or the like. In an injectable formulation, the protein or nucleic acid of the present disclosure can be dissolved in an aqueous solution, preferably a physiologically compatible buffer such as Hanks' solution, Ringer's solution, or physiological saline buffer.

The composition of the present disclosure can take the form of a suspension, a solution, an emulsion, or the like in an oily or aqueous vehicle. Alternatively, the protein or nucleic acid of the present disclosure may be produced in the form of a powder together with a carrier or the like, and an aqueous solution or a suspension may be prepared using sterile water or the like before use. For administration by inhalation, the protein or nucleic acid of the present disclosure can be powdered together with a carrier or the like, and made into a powder mixture together with a suitable base such as lactose or starch. A suppository formulation can be produced by mixing the protein or nucleic acid of the present disclosure with a conventional suppository base such as cocoa butter together with a carrier or the like. Furthermore, the composition for treating cancer of the present disclosure can be encapsulated in a polymer matrix or the like, and formulated as a sustained release formulation.

In a case where the composition of the present disclosure contains a nucleic acid encoding a protein, in one aspect, the nucleic acid can be administered into a living body in the form of mRNA expressed as a protein in the living body, and can be expressed in the circulating blood in the body or near a tumor. The dosage form used for administration in the form of the mRNA is not particularly limited, and a dosage form known to those skilled in the art can be used. For example, mRNA used as the nucleic acid of the present disclosure can be used by being encapsulated in a liposome. The method for forming a liposome is not particularly limited, and a known method known to those skilled in the art can be used. A liposome is a single-layer or multi-layer vesicle and has a membrane portion formed of a lipophilic material and an internal aqueous portion. The aqueous portion is used to contain a polynucleotide material to be delivered to a target site. In one aspect, the liposome can contain one or more cationic lipids, one or more non-cationic lipids, one or more cholesterol-based lipids, and one or more PEG-modified lipids. In one aspect, the one or more cationic lipids can be selected from the group consisting of C12-200, MC3, DLinDMA, DLinkC2DMA, cKK-E12, ICE (imidazole-based), HGT5000, HGT5001, DODAC, DDAB, DMRIE, DOSPA, DOGS, DODAP, DODMA, and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, HGT4003, and combinations thereof.

A concentration of the composition of the present disclosure is appropriately adjusted. For example, in a case where a protein is used, it can be used at a concentration of 1 µg/mL to 50 mg/mL, 10 µg/mL to 50 mg/mL, 20 µg/mL to 50 mg/mL, 30 µg/mL to 50 mg/mL, 40 µg/mL to 50 mg/mL, 50 µg/mL to 50 mg/mL, 60 µg/mL to 50 mg/mL, 70 µg/mL to 50 mg/mL, 80 µg/mL to 50 mg/mL, 90 µg/mL to 50 mg/mL, 0.1 mg/mL to 50 mg/mL, 0.2 mg/mL to 50 mg/mL, 0.3 mg/mL to 50 mg/mL, 0.4 mg/mL to 50 mg/mL, 0.5 mg/mL to 50 mg/mL, 0.6 mg/mL to 50 mg/mL, 0.7 mg/mL to 50 mg/mL, 0.8 mg/mL to 50 mg/mL, 0.9 mg/mL to 50 mg/mL, 1 mg/mL to 50 mg/mL, 2 mg/mL to 50 mg/mL, 3 mg/mL to 50 mg/mL, 4 mg/mL to 50 mg/mL, 5 mg/mL to 50 mg/mL, 6 mg/mL to 50 mg/mL, 7 mg/mL to 50 mg/mL, 8 mg/mL to 50 mg/mL, 9 mg/mL to 50 mg/mL, 10 mg/mL to 50 mg/mL, 20 mg/mL to 50 mg/mL, 30 mg/mL to 50 mg/mL, 40 mg/mL to 50 mg/mL. For example, in the case where a nucleic acid is used, it can be used at a concentration of 20 µg/mL to 30 mg/mL, 30 µg/mL to 30 mg/mL, 40 µg/mL to 30 mg/mL, 50 µg/mL to 30 mg/mL, 60 µg/mL to 30 mg/mL, 70 µg/mL to 30 mg/mL, 80 µg/mL to 30 mg/mL, 90 µg/mL to 30 mg/mL, 100 µg/mL to 30 mg/mL, 200 µg/mL to 30 mg/mL, 300 µg/mL to 30 mg/mL, 400 µg/mL to 30 mg/mL, 500 µg/mL to 30 mg/mL, 600 µg/mL to 30 mg/mL, 700 µg/mL to 30 mg/mL, 800 µg/mL to 30 mg/mL, 900 µg/mL to 30 mg/mL, 1 mg/mL to 30 mg/mL, 2 mg/mL to 30 mg/mL, 3 mg/mL to 30 mg/mL, 4 mg/mL to 30 mg/mL, 5 mg/mL to 30 mg/mL, 6 mg/mL to 30 mg/mL, 7 mg/mL to 30 mg/mL, 8 mg/mL to 30 mg/mL, 9 mg/mL to 30 mg/mL, 10 mg/mL to 30 mg/mL, 20 mg/mL to 30 mg/mL.

In a case where the composition of the present disclosure is used as a reagent, the amount of protein or nucleic acid used can be appropriately adjusted depending on the purpose. For example, in a case where a protein is used, the amount is preferably 1 µg to 500 mg, and in a case where a nucleic acid is used, the amount is preferably 20 µg to 300 µg.

In a case where the composition of the present disclosure is administered to a living body, a dosage of the protein or nucleic acid varies depending on the patient's symptoms, a route of administration, body weight, age, and the like, but is preferably, for example, 1 µg to 500 mg of protein and 20 µg to 300 µg of nucleic acid per day for an adult. Further, when used in combination with another cancer treatment method, the dosage of the composition of the present disclosure can be appropriately adjusted according to the effect of the other treatment method, the patient's condition, and the like. In one aspect, in a case where the composition of the present disclosure and another cancer therapeutic drug are administered in combination, it is preferable to use these dosages at each effective amount or up to 0.01 to 1 times the effective amount. Further, when used in combination with the composition of the present disclosure, a radiation dose in another cancer therapy, for example, radiation therapy, can be reduced to 0.1 to 0.8 times.

The route of administration of the composition of the present disclosure is not particularly limited. The composition of the present disclosure can be administered, for example, but not limited to, by parenteral routes of administration, for example, injections (subcutaneous injection, intravenous injection, intramuscular injection, intraperitoneal injection, etc.), or transdermally, transmucosally, nasally, pulmonarily, orally.

In one aspect, the organism to which the composition of the present disclosure is to be administered is not particularly limited. The subject organism can be selected from primates such as a human and a monkey, mammals such as a dog and a cat, and the like, but is not limited thereto.

### (Method of using composition containing protein having PtdSer binding site of MFG-E8 but not having integrin αᵥβ_{3/5}, α₈β₁ binding site, or nucleic acid encoding the same)

In one aspect, the present disclosure provides a method of using a composition containing a protein that has a PtdSer binding site of MFG-E8 and does not have an integrin αᵥβ_{3/5}, α₈β₁ binding site, or a nucleic acid encoding the same. Typically, the method of the present disclosure uses the composition described above (composition containing a protein that has a PtdSer binding site of MFG-E8 and does not have an integrin αᵥβ_{3/5}, α₈β₁ binding site, or a nucleic acid encoding the same), and the protein is the protein described in (a protein that has a PtdSer binding site of MFG-E8 and does not have an integrin αᵥβ_{3/5}, α₈β₁ binding site).

In one aspect, the method of the present disclosure is a method for activating phagocytosis of phagocytes in vivo or in vitro.

Therefore, in one aspect, the present disclosure provides a method of activating phagocytosis of phagocytes, including a step of bringing a protein that has a PtdSer binding site of MFG-E8 and does not have an integrin αᵥβ_{3/5}, α₈β₁ binding site, into contact with PtdSer of apoptotic cells.

In one aspect, the present disclosure provides a method of activating phagocytosis of phagocytes, including bringing a protein that has a PtdSer binding site of MFG-E8 and does not have an integrin αᵥβ_{3/5}, α₈β₁ binding site, into contact with PtdSer of apoptotic cells, wherein the method uses a composition containing the protein or a nucleic acid encoding the same.

In one aspect, the method of the present disclosure provides a method of activating phagocytosis of phagocytes in a subject having a disease, symptom, or condition requiring activation of phagocytosis of phagocytes, including a step of administering to the subject a composition containing a protein that has a PtdSer binding site of MFG-E8 and does not have an integrin αᵥβ_{3/5}, α₈β₁ binding site, or a nucleic acid encoding the same.

In one aspect, the method of the present disclosure provides a method of treating a disease, symptom, or condition, in a subject having a disease, symptom, or condition requiring activation of phagocytosis of phagocytes, including a step of administering to the subject a composition containing a protein that has a PtdSer binding site of MFG-E8 and does not have an integrin αᵥβ_{3/5}, α₈β₁ binding site, or a nucleic acid encoding the same.

In one aspect, the method of the present disclosure is use of the composition of the present disclosure in treatment of a disease, symptom, or condition requiring activation of phagocytosis of phagocytes.

Therefore, in one aspect, the present disclosure provides the use of a composition containing a protein that has a PtdSer binding site of MFG-E8 and does not have an integrin αᵥβ_{3/5}, α₈β₁ binding site, or a nucleic acid encoding the same, in a method of activating phagocytosis of phagocytes in a subject having a disease, symptom, or condition requiring activation of phagocytosis of phagocytes.

In one aspect, the present disclosure provides the use of a composition containing a protein that has a PtdSer binding site of MFG-E8 and does not have an integrin αᵥβ_{3/5}, α₈β₁ binding site, or a nucleic acid encoding the same, in a method of treating the disease, symptom, or condition in a subject having a disease, symptom, or condition requiring activation of phagocytosis of phagocytes.

In one aspect, the method of the present disclosure is a method of activating an immune response.

Therefore, in one aspect, the method of the present disclosure provides a method of activating an immune response in a subject having a disease, symptom, or condition requiring activation of an immune response, including a step of administering to the subject a composition containing a protein that has a PtdSer binding site of MFG-E8 and does not have an integrin αᵥβ_{3/5}, α₈β₁ binding site, or a nucleic acid encoding the same.

In one aspect, the method of the present disclosure is the use of the composition of the present disclosure in activation of an immune response.

Therefore, in one aspect, the present disclosure provides the use of a composition containing a protein that has a PtdSer binding site of MFG-E8 and does not have an integrin αᵥβ_{3/5}, α₈β₁ binding site, or a nucleic acid encoding the same, in a method of activating an immune response in a subject having a disease, symptom, or condition requiring activation of an immune response.

In one aspect, the method of the present disclosure is a method of treating cancer.

Therefore, in one aspect, the method of the present disclosure provides a method of treating cancer in a subject requiring cancer treatment, including a step of administering to the subject a composition containing a protein that has a PtdSer binding site of MFG-E8 and does not have an integrin αᵥβ_{3/5}, α₈β₁ binding site, or a nucleic acid encoding the same.

In one aspect, the method of the present disclosure is the use of the composition of the present disclosure in a method of treating cancer.

Therefore, in one aspect, the present disclosure provides the use of a composition containing a protein that has a PtdSer binding site of MFG-E8 and does not have an integrin αᵥβ_{3/5}, α₈β₁ binding site, or a nucleic acid encoding the same, in a method of treating cancer in a subject requiring cancer treatment.

The composition used in the method of the present disclosure is used for the configuration, amount used, administration method, route of administration, administration subject, and the like described above (composition containing a protein that has a PtdSer binding site of MFG-E8 and does not have an integrin αᵥβ_{3/5}, α₈β₁ binding site, or a nucleic acid encoding the same). Further, as described above, other cancer therapeutic agents and the like can be used in combination.

### Examples

Hereinafter, the present disclosure will be described in more detail with reference to examples, but these are merely illustrative and do not limit the present disclosure.

### Example 1: Confirmation of cancer therapeutic effect in mouse model

### Materials and methods

Experimental method: A cell line was produced in which a modified mouse MFG-E8 protein with a deletion of an EGF-like domain: C1C2 (Fig. 2B), or a modified mouse MFG-E8 protein in which a portion of RAP (Y38 to L360) was fused to the C-terminus of a modified mouse MFG-E8 protein with a deletion of an EGF-like domain: PStRAP (Fig. 2C) was overexpressed in a mouse fibrosarcoma cell line MCA205(donated by Dr. Steven A. Rosenberg of NIH) that constitutively expresses wild-type MFG-E8.

The amino acid sequences of Y38 to L360 of mouse RAP protein (mRAP) and corresponding amino acid sequences of Y35 to L357 of human RAP protein (hRAP) are shown below.
mRAP (Y38-L360) : (SEQ ID NO: 1) 
YSREKNEPEMAAKRESGEEFRMEKLNQLWEKAKRLHLSPVRLAELHSDLKIQERDELNW
KKLKVEGLDKDGEKEAKLIHNLNVILARYGLDGRKDAQMVHSNALNEDTQDELGDPRLE
KLWHKAKTSGKFSSEELDKLWREFLHYKEKIQEYNVLLDTLSRAEEGYENLLSPSDMAH
IKSDTLISKHSELKDRLRSINQGLDRLRKVSHQGYGSTTEFEEPRVIDLWDLAQSANFT
EKELESFREELKHFEAKIEKHNHYQKQLEISHQKLKHVESIGDPEHISRNKEKYVLLEE
KTKELGYKVKKHLQDLSSRVSRARHNEL
hRAP (Y35-L357) : (SEQ ID NO: 2)

YSREKNQPKPSPKRESGEEFRMEKLNQLWEKAQRLHLPPVRLAELHADLKIQERDELAW
KKLKLDGLDEDGEKEARLIRNLNVILAKYGLDGKKDARQVTSNSLSGTQEDGLDDPRLE
KLWHKAKTSGKFSGEELDKLWREFLHHKEKVHEYNVLLETLSRTEEIHENVISPSDLSD
IKGSVLHSRHTELKEKLRSINQGLDRLRRVSHQGYSTEAEFEEPRVIDLWDLAQSANLT
DKELEAFREELKHFEAKIEKHNHYQKQLEIAHEKLRHAESVGDGERVSRSREKHALLEG
RTKELGYTVKKHLQDLSGRISRARHNEL

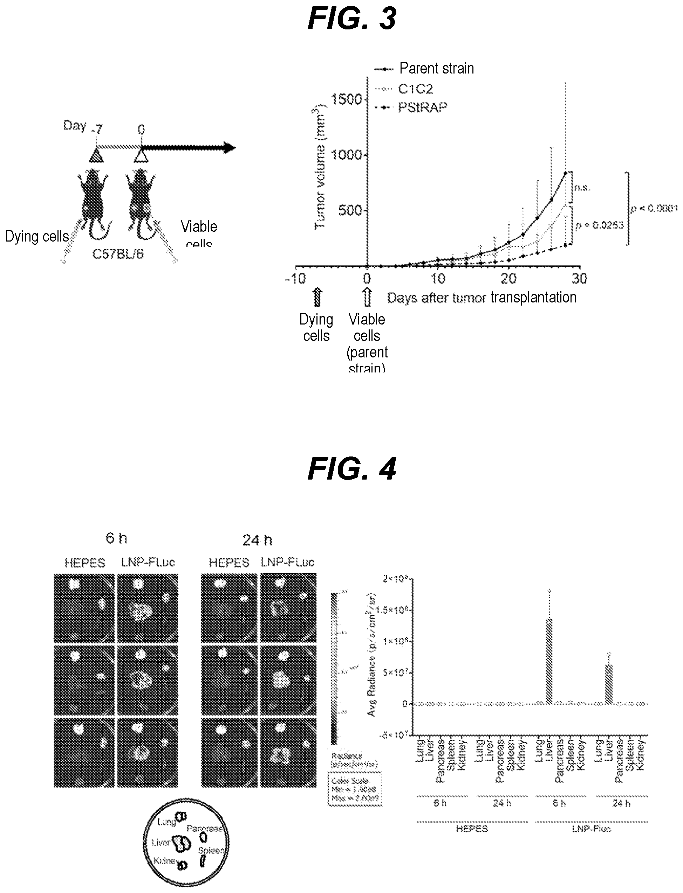

An overview is shown on a left side of Fig. 3. MCA205 parent strain or modified overexpressing strains were treated with mitomycin C to stop proliferation, and 5 x 10⁵ dying cells were subcutaneously administered to a left ventral site of wild-type C57BL/6 mice, respectively (n = 8/group). Seven days after the administration, 5×10⁵ MCA205 parent strains were subcutaneously transplanted to a right ventral site, and tumor growth was measured over time. A tumor growth curve shows a mean value, an error bar shows a standard deviation (SD). A statistical analysis was performed using a 2-way ANOVA followed by Tukey's test as a post-hoc test, and p < 0.05 was considered significant.

Results:
The results are shown in a graph on a right side of Fig. 3. Complete rejection rates of transplanted tumor cells in groups to which the parent strain, the C1C2-expressing strain, and the PStRAP-expressing strain were administered as dying cells were 1/8, 2/8, and 3/8, respectively. In the group to which the PStRAP-expressing strain was administered, delay in tumor growth was clearly observed as compared with the group to which the parent strain or the C1C2-expressing strain was administered. In the C1C2-expressing strain, no statistically significant difference was detected in the tumor growth curve compared with the parent strain, but the number of complete rejections was higher.

Discussion: The results of this experiment show that a protein that has a PtdSer binding site of MFG-E8 and does not have an integrin αᵥβ_{3/5}, α₈β₁ binding site according to the present disclosure enhances the immune response against cancer cells through regulation of the phagocytic pathway of phagocytes, and has an excellent cancer therapeutic effect. In particular, the modified MFG-E8 protein in which a portion of RAP (Y38 to L360) was fused showed an extremely excellent cancer therapeutic effect.

### Example 2: Properties of LNP

### Materials and methods

As an LNP (lipid nanoparticle), commercially available ALC-0315 was used. mRNA expressing firefly luciferase (Fluc) was encapsulated in the LNP, and the LNP was intravenously administered to wild-type mice. As a negative control group, HEPES, used as a solvent, was intravenously administered. Luciferin was administered 6 hours and 24 hours after administration, followed by euthanasia. Each organ was collected, and luminescence from a luciferase protein was detected using an IVIS imaging system. Three mice were used in each group.

Results: The results of detecting luminescence from the luciferase protein are shown in Fig. 4. It was found that the LNP was preferentially delivered to the liver 6 hours after administration, and the mRNA was translated and expressed as a protein. The amount of protein had decreased 24 hours after administration. In the graph on the right side of Fig. 4, the error bar indicates a standard deviation (SD).

### Example 3: Evaluation of liver dysfunction induced by LNP administration

### Materials and methods

LNP-mRNA prepared in the same manner as in Example 2 was intravenously administered to wild-type mice. As a negative control group, HEPES, used as a solvent, was intravenously administered. Serial blood collection was performed 6, 24, and 48 hours after administration, and ALT and AST concentrations in the blood were measured. Four mice were used in each group.

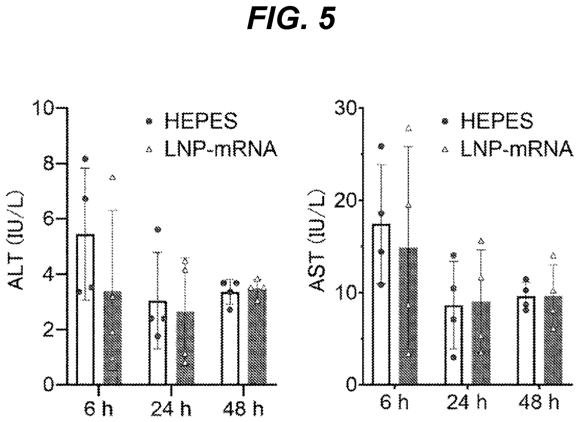

Results:
The administered LNP accumulated in the liver 6 hours after administration, but no liver dysfunction was observed (Fig. 5, the error bar represents the SD.).

### Example 4: Evaluation of binding specificity of PStRAP to cell membrane lipids

### Materials and methods

Hepatocytes were isolated from wild-type mice, PStRAP mRNA was transfected in vitro, and PStRAP secreted into a supernatant after 6 hours of culture was used. Binding of PStRAP was evaluated using a membrane lipid strip (P-6002, ECHELON BIOSCIENCES) with 15 types of lipids spotted onto a hydrophobic membrane.

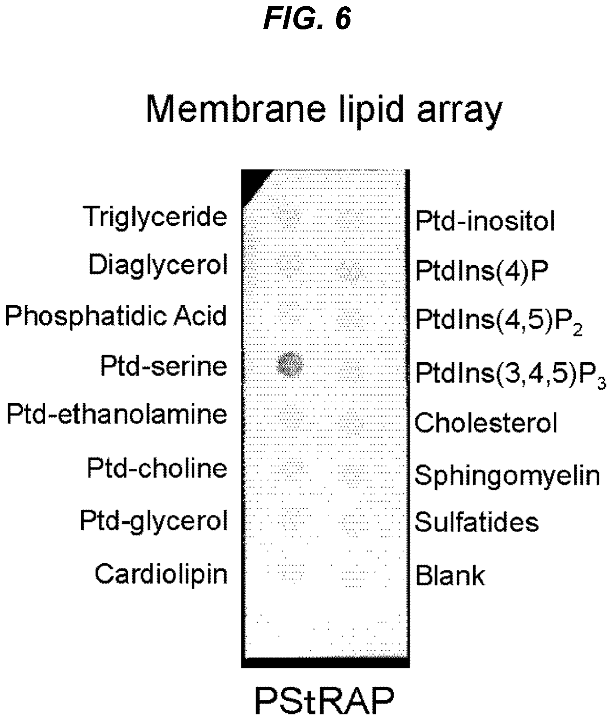

Results:
The results are shown in Fig. 6. It was found that PStRAP specifically binds to phosphatidylserine (Ptd-serine).

### Example 5: Measurement of amount of PStRAP in serum after LNP-PStRAP administration

### Materials and methods

mRNA expressing PStRAP (with a Flag-tag added to the C-terminus) was encapsulated in the LNP (ALC-0315 described above), and the LNP was intravenously administered to wild-type mice. As a negative control group, HEPES, used as a solvent, was intravenously administered. Serial blood collection was performed 6, 24, 72, and 120 hours after administration, and serum was obtained. The amount of PStRAP contained in the serum was semi-quantified by an ELISA method targeting the Flag-tag. Four mice were used in each group. For the semi-quantitative results, a significance test by Student t-test was performed (*: p < 0.05, ***: p < 0.0001).

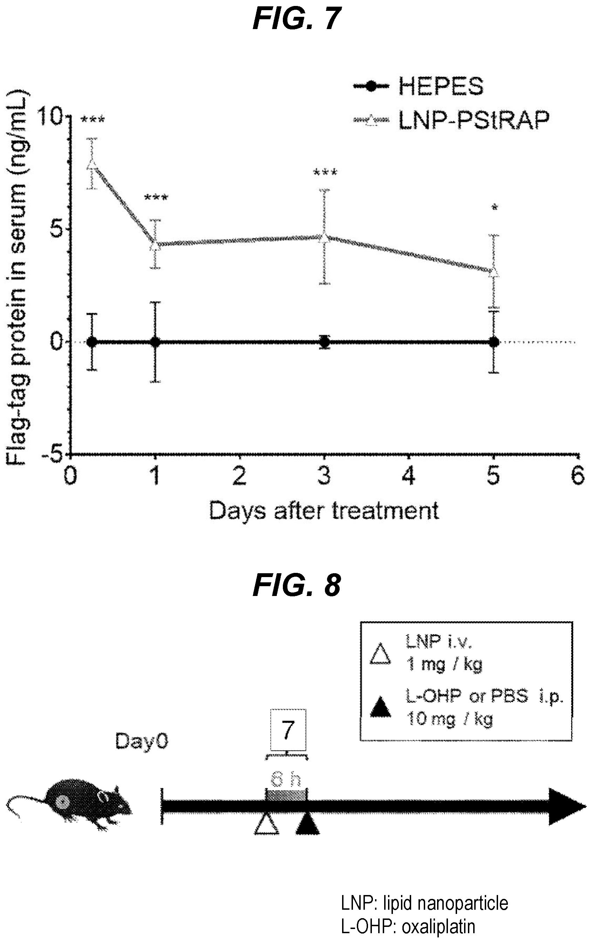

Results:
The results are shown in Fig. 7 (the error bar indicates the SD). It was found that PStRAP in serum was detected at the highest level 6 hours after administration and decreased over time.

### Example 6: Confirmation of antitumor effect by combination use of LNP-PStRAP and anticancer agent

### Materials and methods

The overview is shown in Fig. 8. A mouse fibrosarcoma cell line MCA205 was subcutaneously transplanted into wild-type mice to create a tumor-bearing mouse model. Seven days after tumor transplantation, mRNA expressing PStRAP was encapsulated in the LNP (ALC-0315 described above), and the LNP was intravenously administered. As a negative control group, an empty LNP (Empty) containing no mRNA was intravenously administered. The anticancer agent oxaliplatin (L-OHP) or PBS as a negative control was intraperitoneally administered 6 hours after LNP administration (Fig. 8). Thereafter, a tumor diameter was measured and a tumor volume was calculated to measure a tumor growth rate. Six mice were used in each group. The measurement results were subjected to a significance test by 2-way ANOVA followed by Tukey's test.

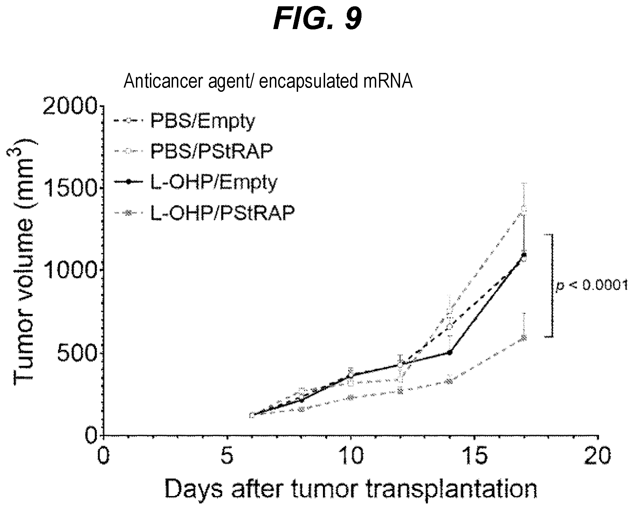

Results:
The results are shown in Fig. 9 (the error bar indicates the SD). A significant antitumor effect was observed in the oxaliplatin and LNP-PStRAP combination group. The present results indicate that particularly excellent anticancer effects can be obtained by using the modified MFG-E8 protein of the present disclosure or a nucleic acid encoding the same in combination with other anticancer therapies.

### Industrial Applicability

The present disclosure has an extremely high industrial utility value by providing a new cancer therapeutic agent and a cancer treatment method, and a phagocyte activator and an activation method, an immune response activator and an activation method related thereto.

## Claims

1. A protein that has a phosphatidylserine (PtdSer) binding site of milk fat globule-EGF factor 8 (MFG-E8) and does not have an integrin αᵥβ_{3/5}, α₈β₁ binding site.

2. The protein according to claim 1, wherein the protein has a C1C2 domain of MFG-E8 or a portion thereof as a PtdSer binding site.

3. The protein according to claim 1, wherein the protein does not have a RGD motif (Arg-Gly-Asp integrin binding motif) of MFG-E8 or a portion thereof as an integrin αᵥβ_{3/5}, α₈β₁ binding site.

4. The protein according to claim 1, wherein the protein does not have an EGF-like domain (epidermal growth factor like domain) of MFG-E8 or a portion thereof as an integrin αᵥβ_{3/5}, α₈β₁ binding site.

5. The protein according to claim 1, wherein the protein further has a site that binds to a receptor that is different from integrin αᵥβ_{3/5} or integrin α₈β₁ on phagocytes.

6. The protein according to claim 5, wherein the receptor on phagocytes is a receptor that activates phagocytosis of phagocytes.

7. The protein according to claim 6, wherein the receptor that activates phagocytosis of the phagocytes is CD91/LRP1.

8. The protein according to claim 5, wherein the site that binds to the receptor on phagocytes is a receptor-associated protein (RAP) or a portion thereof.

9. A nucleic acid encoding the protein according to any one of claims 1 to 8.

10. A composition for activating phagocytosis of phagocytes, comprising the protein according to any one of claims 1 to 8 or a nucleic acid encoding the same.

11. A composition for activating an immune response, comprising the protein according to any one of claims 1 to 8 or a nucleic acid encoding the same.

12. A composition for treating cancer, comprising the protein according to any one of claims 1 to 8 or a nucleic acid encoding the same.

13. The composition for treating cancer according to claim 12, for use in combination with an anticancer therapy exhibiting a cytocidal effect.

14. The composition for treating cancer according to claim 12, for use in combination with an anticancer therapy using an immune checkpoint inhibitor.
